# FASCICULE DE BREVET EUROPEEN

(11) **EP 1 521 586 B1**
(45) Date de publication et mention de la délivrance du brevet: **04.04.2007**
(21) Numéro de dépôt: 03763945.7
(22) Date de dépôt: 09.07.2003
(51) Int. Cl.: A61K 36/28, A61K 9/107, A61P 17/10

(54) **UTILISATION D' UN EXTRAIT DE CHRYSANTHELLUM INDICUM POUR LE TRAITEMENT DE L'ACNE ROSACEE**
VERWENDUNG VON EINEM CHRYSANTHELLUM INDICUM EXTRAKT ZUR BEHANDLUNG DER AKNE ROSACEA
USE OF A CHRYSANTHELLUM INDICUM EXTRACT FOR THE TREATMENT OF ACNE ROSACEA

(30) Priorité: 11.07.2002 FR 0208753
(43) Date de publication de la demande: 13.04.2005
(73) Titulaire: Laboratoire Sicobel, 69570 Dardilly (FR)
(72) Inventeur: FOUCHERE, Nicolas, F-69110 Sainte Foy Les Lyon (FR)
(74) Mandataire: Tripoz, Inès
(86) Numéro de dépôt international: PCT/FR2003/002147
(87) Numéro de publication internationale: WO 2004/006943

(56) Documents cités:
- WO-A-98/20851
- FR-A- 2 276 060
- FR-A- 2 378 044
- FR-A- 2 456 116
- FR-A- 2 618 071

## Description

La présente invention concerne une nouvelle application thérapeutique en dermatologie d'une composition comprenant un extrait de Chrysanthellum.

Elle a plus particulièrement pour objet l'utilisation d'un extrait de Chrysanthellum indicum pour la préparation d'une composition topique destinée au traitement de l'acné rosacée.

Cette affection cutanée est caractérisée par une rougeur de la face et du décolleté liée à la stase capillaire et par des réactions inflammatoires et un état oedémateux avec ou sans microvaisseaux apparents.

Outre les problèmes de douleur et de gêne provoqués par l'état inflammatoire et oedémateux, la localisation sur la face et le décolleté ajoute une composante inesthétique à la gravité de cette affection.

Dans l'acné rosacée, on observe une éruption caractérisée par un érythème facial, des télangiectasies et des papulo-pustules, localisées le plus souvent aux sillons nasogéniens, aux pommettes, au nez et au menton.

De nombreuses préparations dermatologiques ont été utilisées dans le traitement de cette affection cutanée, sans réelle efficacité.

Dans le cas de l'acné rosacée, des médicaments à activité antibactérienne et anti-inflammatoire sont même utilisés comme le métronidazole (Dénomination Commune Internationale).

Le chrysanthellum est une plante sub-tropicale appartenant à la famille des Hélianthées, cette plante se rencontre en Afrique Centrale et en Amérique du Sud.

Les variétés de Chrysanthellum étudiées pour leurs applications phytopharmaceutiques sont les variétés chrysanthellum americanum vatke, chrysanthellum procumbens pers et chrysanthellum indicum, afro-américanum sous forme d'extraits aqueux ou hydro-alcoolique.

Ces différents extraits se différencient par leur composition qui varient en fonction des méthodes d'extraction mises en oeuvre.

On retrouve cependant de manière prépondérante des dérivés flavonoïdes et des saponines.

On connaît de BSM-FR 979 et de FR-A-2 096 902 des médicaments obtenus à partir d'extraits aqueux ou hydro-alcooliques de Chrysanthellum Americanum Vatke destinés au traitement des troubles intestinaux associés ou non a des troubles hépatiques.

On connaît de EP-A-317453 des compositions médicamenteuses, utilisées dans le traitement de la lithiase cystinique, des artérites et de l'insuffisance veineuse des membres inférieurs, comprenant en association des flavonoïdes et des saponines extraits de Chrysanthellum et leur procédé d'extraction.

Des compositions cosmétiques et dermatologiques présentant une action anti-inflammatoire, régulatrice de la microcirculation cutanée et détergente douce, ont été décrites dans FR2618071.

WO 98 20851 décrit une composition comportant de 0,0001% à 0,05 % d'équivalents d'extraits secs de Chrysanthellum Indicum, ayant une action anti-radicalaire et anti-lipoperoxydante, utilisée pour la prévention du vieillissement cutané. La composition peut être administrée sous une forme utilisable par voie topique.

FR-A-2 618 071 décrit des compositions cosmétiques et dermatologiques contenant entre 1 % et 10 % en poids d'extrait sec de Chrysanthellum Americanum, Procumbens, Indicum ou Afro-Americanum. Les extraits utilisés ont une action anti-inflammatoire, régulatrice de la micro circulation cutanée.

FR-A-2 276 060 décrit des composition obtenues à partir de variétés de Chrysanthellum. Les produits peuvent être utilisés sous forme de pommades, gels, crèmes. Les produits diminuent la perméabilité et accroissent la résistance capillaire. Ils peuvent être utilisés dans les vasculopathies caractérisées par une fragilisation de la paroi, dans les affections à hauts risques vasculaires, par ex. les oedèmes, purpuras.

FR-A-2 378 044 décrit des extraits végétaux de Chrysanthellum ayant une action anti-inflammatoire locale et des effets capillaroprotecteurs, pouvant notamment être utilisés en phlébologie, dans le traitement des oedèmes, purpuras... Ils peuvent être administrés sous forme de pommades, gels, crèmes.

FR-A-2 456 116 décrit une saponine extraite de Chrysanthellum, son effet thérapeutique sur la résistance et la perméabilité capillaire et son utilisation par ex. en phlébologie, dans le traitement des oedèmes, purpuras etc. Elle peut être administrée sous forme de gel, crème, pommade.

La présente invention a pour objet l'utilisation d'un extrait de Chrysanthellum Indicum, pour la préparation d'une composition topique destinée au traitement de l'acné rosacée.

Selon un mode préféré de réalisation de l'invention les compositions topiques comprennent entre 0,01% à 2 % d'extrait sec de Chrysanthellum Indicum, auquel peut être éventuellement associé 0,3% à 0,5% d'enoxolone.

L'extrait de Chrysanthellum Indicum utilisé est obtenu par mise en oeuvre d'un procédé d'extraction classiquement utilisé pour traiter des plantes.

On soumet par exemple l'ensemble de la plante, fraîche ou sèche à un broyage mécanique jusqu'à obtention d'une poudre grossière Cette poudre est ensuite soumise à une étape de macération pendant 10 à 24 heures à température ambiante dans de l'eau ou dans un mélange 50/50 eau/éthanol ou eau/méthanol, puis on fait subir à l'ensemble obtenu une lixiviation en continu à 50°C dans un percolateur avec un mélange 50/50 eau/éthanol ou eau/méthanol, le rapport plante solvant étant de 1 /5 (P/V).

L'extrait obtenu, est ensuite soumis à des lavages liquide/liquide à l'aide d'un solvant organique non polaire comme le di ou le tri-chlorométhane. Après concentration et séchage on obtient un liquide sirupeux qui évaporé jusqu'à siccité donne une poudre jaune brun soluble dans l'eau et très soluble dans les alcools.

L'extrait sec obtenu contient des saponines Chrisanthelline A et B, des acides phénols et des flavonoïdes.

La teneur moyenne en flavonoïdes de l'extrait sec de Chrysanthellum utilisé est comprise entre 7 et 9%.

La solution de Chrysanthellum indicum incluse dans la composition topique selon l'invention, destinée au traitement de l'érythrocouperose est obtenue soit par solubilisation de l'extrait sec dans de l'eau suivie d'une phase de décantation, soit par solubilisation hydroalcoolique de l'extrait sec, dans cette phase de solubilisation des solvants comme le propylène glycol ou le butylène glycol peuvent être utilisés, suivie d'une phase de décantation. Cette phase de décantation est destinée à l'élimination des mucilages pour permettre le titrage de ladite solution en flavonoïdes.

Les solutions utilisées pour la préparation des compositions selon l'invention sont des solutions à 2% d'extrait sec.

Parmi les extraits végétaux pouvant être utilisés en association avec l'extrait de Chrysanthellum indicum on peut citer l'enoxolone sous peut citer l'enoxolone sous forme de poudre obtenue à partir de Glyccyrhyza Glabra, à des concentrations de 0,3 à 0,5% en poids, des extraits de Mimosa Tenuiflora à des concentrations de 2 à 4% en poids, des extraits de Crataegus Oxyaxantha à des concentrations de 0,5 à 1% en poids, un extrait de Ruscus Aculeatus à des concentrations de 0,5 à 1 % en poids, une solution de Ranunculus Ficaria à des concentrations de 0,5 à 1 % en poids une solution du complexe Cupressus Sempervirens/Arnica Montana/Polygonatum Multifolium à des concentrations de 3 à 5% en poids.

Les compositions utilisables selon l'invention peuvent en outre comprendre un filtre solaire minéral pour protéger la peau contre les rayons U.V. ; ce filtre est ajouté au mélange dans des proportions qui varient de 1 à 2% en poids.

Dans un mode de réalisation préféré les compositions utilisables selon l'invention comportent en outre un agent épaississant choisi parmi le carbomer, la gomme scletorium et la gomme xanthane dans les proportions avantageuses suivantes : Carbomer 0,1 à 0, 2%, gomme scletorium 0,2 à 0,4% gomme xanthane 0,2 à 0, 5%.

La présente invention concerne également l'utilisation d'un extrait de Chrysanthellum Indicum pour la préparation d'une composition topique destinée au traitement de l'acné rosacée, caractérisée en ce que la teneur en extrait sec de Chrysanthellum Indicum est comprise entre 0,01 et 2 % en poids.

Elle concerne également ladite utilisation caractérisée en ce que les compositions selon l'invention comprennent en outre un autre extrait végétal seul ou en association choisi parmi l'enoxolone obtenue à partir de Glyccyrhyza Glabra, des extraits de Mimosa Tenuiflora des extraits de Crataegus Oxyaxantha, un extrait de Ruscus Aculeatus, une solution de Ranunculus Ficaria, une solution du complexe Cupressus Sempervirens/Arnica Montana/Polygonatum Multifolium à des concentrations comprises entre 0,3 et 5 % en poids.

La présente invention concerne également une composition topique destinée au traitement de l'acné rosacée, comprenant de 0,01 à 0,02 % d'extrait sec de Chrysanthellum Indicum, caractérisée en ce qu'elle comprend en outre en association avec l'extrait de chrysanthellum Indicum de 0,3 à 0, 5% d'enoxolone.

Différentes compositions utilisables selon l'invention sont décrites ci après :
FORMULE A : spray ou lotion

| | |
|---|---|
| 0,5 à 1% | solution de Chrysanthellum Indicum 0 2 % |
| 0,5 % | Polysorbate 20 |
| 0,3 % | gomme xanthane |
| 0,20 % | benzoate de sodium |
| 0,15 % | hydroxide de sodum ne solution à 10% |
| 0,10 % | acide sorbique |
| 0,08 % | menthol |
| Q.S.P. | eau déminéralisée. |

FORMULE B : émulsion

| | |
|---|---|
| 8% | hexyldecanol hexyldecyl laurate |
| 5 % | Cinnamonum zeylanicum |
| 5 % | Arnica montana, |
| | Cupressus sempervirens, |
| | Polygonatum multifolium |
| 5 % | sorbitan stearate sucrose cocoate |
| 5 % | butylène glycol |
| 4% | Mimosa tenuiflora |
| 2% | Borago officinalis |
| 2% | Prunus amygdalus dulcis |
| 2% | glycerin |
| 2% | dicaprylyl carbonate |
| 2% | lecithin |
| 1,5 % | Butyrospermum parkii |
| 1,5 % | dimethicone |
| 0,5 à 1 % | solution de Chrysanthellum Indicum à 2 % |
| O,1 % | Titanium dioxide et Mica |
| 1 % | myristyl myristate |
| 1 % | hydroxide de sodium |
| 0,3 à 0,5 % | acide glycyrrhetinique |
| 0,2 à 0, 4 % | gomme sclertorium |
| 0,2 à 0, 4 % | oleate de glycerol, tocopherol, |
| | ascorbyl palmitate et acide citrique |
| 0,2 % | methylparaben |
| 0,1 à 0,15 % | carbomer |
| 0,1 % | parfum |
| 0,05 % | disodium EDTA |
| Q.S.P. | eau déminéralisée |

L'invention sera mieux comprise à la lecture des tests ayant permis de mettre en évidence les propriétés des formulations selon l'invention.

### Evaluation in vivo :

- Scorage de l'érythrose:
   Le scorage de l'érythrose est effectué quotidiennement par un dermatologue selon deux échelles, une échelle d'intensité allant de « aucune » à « très sévère » et une échelle d'étendue allant de 0 à 100 %, pendant 56 jours avec application biquotidienne des formulations selon l'invention.
   Une diminution significative selon les deux échelles est observée après application quotidienne d'une composition selon l'invention.
- Mesure de la microcirculation cutanée :
   Les mesures ont effectuées à l'aide d'un laser-scanner Imager LISCA sur une zone de 1 cm² sur des sujets atteints d'érythrocouperose.
   Cette méthode non invasive et directe permet la quantification in vivo de l'effet de produits sur la microcirculation cutanée superficielle par mesure du volume de perfusion.
   Des mesures sont effectuées à t 0, puis après 4 semaines, après 8 semaines et après 12 semaines d'application quotidienne de compositions selon l'invention.
   On obtient en moyenne une diminution de 20 % du même volume de perfusion et donc une diminution de la stase capillaire.

Mesure de l'effet anti-inflammatoire :
a) Effet anti-inflammatoire après érythème actinique par mesure au chromamètre Minolta CR 300.
La mesure est effectuée sur une zone ayant subi une irradiation U.V. de 1 DEM.

| N ° formule | Teneur en solution à 2 % d'extrait de Chrysanthellum | Enoxo Ione | Effet |
|---|---|---|---|
| 0 | 0 | 0,3 | 0 |
| 1 | 0,1 | 0 | + |
| 2 | 0,5 | 0 | + + + |
| 3 | 0,5 | 0,3 | + + + |
| 4 | 1 | 0,5 | + + |

La teneur en Chrysanthellum est exprimée en % d'une solution à 2 % d'extrait sec de Chrysanthellum.
L'effet est quantifié + faible effet et + + + effet significatif.
b) Effet anti-inflammatoire après irritation au lauryl sufate (IEC)
Etude conduite sur 16 volontaires, après application de 2 µl/cm² en 3 applications.

| N° formule | Teneur en solution à 2 % d'extrait de Chrysanthellum | Enoxolone | Effet |
|---|---|---|---|
| A | 0,5 | 0,3 | + + |
| B | 0,5 | 0,5 | + |
| C | 1 | 0 | + + + |

L'effet est quantifié + faible effet et + + + effet significatif.

On observe avec les compositions selon l'invention une action anti-inflammatoire significative lorsque la teneur en solution à 2 % d'extrait de Chrysanthellum est comprise entre 0,5 et 1%, soit lorsque la teneur en extrait sec de Chrysanthellum est comprise entre 0,01 et 0,02 %.

### Etude clinique contre Placebo dans le traitement de l'acné rosacée :

Un crème contenant 1 % d'extrait de Chrysanthellum Indicum a été testée dans une étude clinique incluant 246 patients présentant une acné rosacée modérée. 125 patient ont reçu une crème contenant l'extrait de Chrysanthellum Indicum et 121 patients une crème Placebo.

Le nombre d'érythème, la surface des érythèmes, la gravité des lésions et l'efficacité finale ont été déterminés par des investigateurs cliniques.

La crème a été appliquée sur le visage des patients deux fois par jour pendant une péride de douze semaines.

Les patients ont été examinés toutes les quatre semaines.

La crème contenant un extrait de Chrysanthellum Indicum a permis d'obtenir une efficaité significative et une bonne tolérance sur l'érythème et l'acné rosacée modérée, on a en effet observé une amélioration significative sur la gravité de l'érythème et des atteintes cutanées en général par rapport au Placebo.

Les compositions présentent en outre une bonne tolérance cutanée et un effet marqué sur le degré d'hydratation avec une bonne rémanence pendant 8 heures.

## Revendications

1. Utilisation d'un extrait de Chrysanthellum Indicum, pour la préparation d'une composition topique destinée au traitement de l'acné rosacée.

2. Utilisation selon la revendication 1, **caractérisée en ce que** la teneur en extrait sec de Chrysanthellum Indicum est comprise entre 0,01 et 2 % en poids.

3. Utilisation selon l'un quelconque des revendciations précédentes **caractérisée en ce que** les compositions selon l'invention comprennent en outre un filtre solaire minéral dans des proportions de 1 à 2 % en poids.

4. Utilisation selon l'un quelconque des revendications précédentes **caractérisée en ce que** les compositions selon l'invention comprennent en outre un agent épaississant choisi parmi le carbomer, la gomme scletorium ou la gomme xanthane.

5. Utilisation selon l'un quelconque des revendications précédentes **caractérisée en ce que** les compositions selon l'invention comprennent en outre un autre extrait végétal seul ou en association choisi parmi l'enoxolone obtenue à partir de Glyccyrhyza Glabra, des extraits de Mimosa Tenuiflora des extraits de Crataegus Oxyaxantha, un extrait de Ruscus Aculeatus, une solution de Ranunculus Ficaria, une solution du complexe Cupressus Sempervirens/Arnica Montana/Polygonatum Multifolium à des concentrations comprises entre 0,3 et 5 % en poids.

6. Composition topique destinée au traitement de l'acné rosacée, comprenant de 0,01 à 0.02 % d'extrait sec de Chrysanthellum Indicum, **caractérisée en ce qu'**elle comprend en outre en association avec l'extrait de chrysanthellum Indicum de 0,3 à 0, 5% d'enoxolone.

## Claims

1. Use of a Chrysanthellum indicum extract, for the preparation of a topical composition for use in the treatment of acne rosacea.

2. Use according to Claim 1, **characterized in that** the content on a dry extract basis of Chrysanthellum indicum is between 0.01% and 2% by weight.

3. Use according to either one of the preceding claims, **characterized in that** the compositions according to the invention also comprise an inorganic sunscreen in proportions of from 1% to 2% by weight -

4. Use according to any one of the preceding claims, **characterized in that** the compositions according to the invention also comprise a thickener chosen from carbomer, sclerotium gum or xanthan gum.

5. Use according to any one of the preceding claims, **characterized in that** the compositions according to the invention also comprise another plant extract, alone or in combination, chosen from enoxolone obtained from Glycyrrhyza glabra, Mimosa tenuiflora extracts, Crataegus oxyaxantha extracts, a Ruscus aculeatus extract, a solution of Ranunculus ficaria, and a solution of the complex Cupressus sempervirens/Arnica Montana/Polygonatum multifolium at concentrations of between 0.3% and 5% by weight.

6. Topical composition for use in the treatment of acne rosacea, comprising from 0.01% to 0.02% of dry extract of Chrysanthellum indicum, **characterized in that** it also comprises, in combination with the Chrysanthellum indicum from 0.3% to 0.5% of enoxolone.

## Patentansprüche

1. Verwendung eines Chrysanthellum-Indicum-Extrakts zur Herstellung einer topischen Zusammensetzung zur Behandlung von Rosacea.

2. Verwendung nach Anspruch 1, **dadurch gekennzeichnet, daß** der Gehalt an Chrysanthellum-Indicum-Trockenextrakt zwischen 0,01 und 2 Gew.-% liegt.

3. Verwendung nach einem der vorhergehenden Ansprüche, **dadurch gekennzeichnet, daß** die erfindungsgemäßen Zusammensetzungen außerdem ein anorganisches Lichtschutzmittel in Anteilen von 1 bis 2 Gew.-% enthalten.

4. Verwendung nach einem der vorhergehenden Ansprüche, **dadurch gekennzeichnet, daß** die erfindungsgemäßen Zusammensetzungen außerdem ein unter Carbomer, Sclerotium-Gummi oder XanthanGummi ausgewähltes Verdickungsmittel enthalten.

5. Verwendung nach einem der vorhergehenden Ansprüche, **dadurch gekennzeichnet, daß** die erfindungsgemäßen Zusammensetzungen außerdem ein unter aus Glyccyrhyza Glabra erhaltenem Enoxolon, Mimose-Tenuiflora-Extrakten, Crataegus-Oxyaxantha-Extrakten, Ruscus-Aculeatus-Extrakt, Ranunculus-Ficaria-Lösung und einer Lösung des Komplexes Cupressus Sempervirens/Arnica Montana/Polygonatum Multifolium ausgewähltes anderes Pflanzenextrakt alleine oder in Kombination in Konzentrationen zwischen 0,3 und 5 Gew.-% enthalten.

6. Topische Zusammensetzung zur Behandlung von Rosacea, enthaltend 0,01 bis 0,02% Chrysanthellum-Indicum-Trockenextrakt, **dadurch gekennzeichnet, daß** sie außerdem in Kombination mit dem Chrysanthellum-Indicum-Extrakt 0,3 bis 0,5% Enoxolon enthält.
